# EUROPEAN PATENT SPECIFICATION

(11) **EP 0 799 027 B1**
(45) Date of publication and mention of the grant of the patent: **28.02.2001**
(21) Application number: 95938532.9
(22) Date of filing: 04.12.1995
(51) Int. Cl.: A61K 9/127, A61K 38/21

(54) **LIPOSOMAL INTERFERON HYBRID COMPOSITIONS**
LIPOSOMZUBEREITUNGEN ENTHALTEND HYBRID-INTERFERON
COMPOSITIONS DE LIPOSOMES CONTENANT UN INTERFERON HYBRIDE

(30) Priority: 08.12.1994 GB 9426484
(43) Date of publication of application: 08.10.1997
(73) Proprietor: Novartis AG, 4058 Basel (CH); Novartis-Erfindungen Verwaltungsgesellschaft m.b.H., 1235 Wien (AT)
(72) Inventor: TAYLOR, Peter William, West Sussex RH14 9HF (GB); AKHLAQ, Mohammed, Crawley West Sussex RH11 8LY (GB)
(86) International application number: GB9502821
(87) International publication number: WO9617596

(56) References cited:
- EP-A- 0 009 842
- EP-A- 0 331 635
- WO-A-95/20379
- US-A- 4 855 090
- US-A- 4 997 652
- US-A- 5 023 087
- US-A- 5 077 057
- Journal of Interferon Research, Volume 2, No. 1, 1982, Deborah A. Eppstein, "Altered Pharmacologic Properties of Liposome-Associated Human Interferon-Alpha. II."
- Journal of Interferon Research, Volume 10, 1990, Diana M. Smith et al, "Antiviral and Antiproliferative Properties of Liposome-Associated Human Interferon-Y"

## Description

This invention relates to liposomal pharmaceutical compositions containing an α-interferon hybrid, particularly for the treatment of viral liver disease.

Recent clinical trials with interferons have demonstrated their usefulness in the treatment of various diseases, particularly as antiproliferative agents in the treatment of various kinds of cancer and as antiviral agents in the treatment of, for example, hepatitis and Karposi's sarcoma. However, when administered therapeutically via the circulation system, they can give rise to various toxic side effects. Additionally, recombinant interferons frequently elicit the production of neutralising antibodies. It has been proposed to overcome these disadvantages by encapsulating the interferon in liposomes.

Journal of Interferon Research, vol. 2, no. 1, 1982, pp. 117-125 discloses liposomes having a lipid composition of EPC/Chol/PS = 4:5:1 and further comprising human interferon-alpha. An α-interferon B/D hybrid is, however, not specifically mentioned therein.

Recombinant human α-interferon B/D hybrids are of particular interest as antitumour and antiviral agents. In EP 0 331 635 there is disclosed a pharmaceutical composition for the treatment of bladder cancer comprising α-interferon BBDD or BBDB hybrid contained in liposomes formed from palmitoyl-(9-cis-octadecenoyl)-3-sn-phosphatidylchloline (POPC) and 1,2-di-(9-cis-octadecenoyl)-3-sn-phosphatidyl-(S)-serine (OOPS).

There is a need for liposomal α-interferon hybrid compositions having greater stability, i.e. less tendency to leak the protein, in particular for liposomal compositions which are capable of directing an α-interferon hybrid in protected form to liver cells as the target cell population, particularly in the treatment of viral liver disease.

It has now been found that liposomes containing an α-interferon hybrid entrapped in a particular combination of lipids have good stability, with retention of the antiviral activity of the α-interferon hybrid, and show a high capacity to associate with liver cells.

Accordingly, the present invention provides in one aspect a pharmaceutical composition comprising an α-interferon B/D hybrid contained in liposomes formed from a lipid mixture comprising 50 to 75 mol % neutral phospholipid, 20 to 40 mol % cholesterol and 5 to 10 mol % charged phospholipid.

In another aspect the present invention provides the use of an α-interferon hybrid contained in liposomes as hereinbefore defined in the preparation of a medicament for the treatment of viral liver disease.

The α-interferon hybrid is an α-interferon B/D hybrid. The preparation of such hybrids is described in US 4 414 150 and EP 0 205 404. An especially preferred hybrid is α-interferon BDBB, the preparation of which is described in EP 0 205 404.

The lipid component of the liposomes, i.e. the lipid mixture as hereinbefore defined, preferably has a phase transition temperature of 20 to 30°C. The major component of the lipid mixture is preferably the neutral phospholipid component, which is preferably present in an amount of at least 50 mol % of the lipid mixture. In order for the lipid membrane to have a phase transition temperature within the preferred range of 20 to 30°C, the neutral phospholipid component, which preferably comprises one or more phosphatidyl cholines, should preferably have a phase transition temperature of 20 to 30°C. This can be achieved by using, as the neutral phospholipid component, (a) a single phospholipid, preferably a phosphatidylcholine such as dimyristoyl phosphatidylcholine or 1-myristoyl-2-palmitoyl-phosphatidylcholine having a phase transition temperature within this range, (b) a mixture of two or more phospholipids, preferably phosphatidylcholines such as those mentioned hereinbefore, at least one of which has a phase transition temperature within this range, or (c) a mixture of a phospholipid, preferably a phosphatidylcholine, having a phase transition temperature higher than 30°C with a phospholipid, preferably a phosphatidylcholine, having a phase transition temperature below 20°C.

In a preferred embodiment of the invention, the neutral phospholipid is dimyristoyl phosphatidylcholine or a mixture thereof with another neutral phosphatidylcholine, said mixture having a phase transition temperature of 20 to 30°C. In especially preferred embodiments, dimyristoyl phosphatidylcholine is used as the neutral phospholipid component.

The charged phospholipid(s) is (are) preferably negatively charged. Suitable negatively charged phospholipids include phosphatidylserines such as dimyristoyl phosphatidylserine, dipalmitoyl phosphatidylserine, or dioleoyl phosphatidylserine, phosphatidylglycerols, phosphatidic acids and phosphatidylethanolamines. Phosphatidylserines are preferred, especially dioleoyl phosphatidylserine.

The lipid mixture as hereinbefore defined preferably comprises 50 to 75 mol % neutral phospholipid, 20 to 40 mol % cholesterol and 5 to 10 mol % charged phospholipid, more preferably 55 to 70 mol % neutral phospholipid, 25 to 35 mol % cholesterol and 5 to 10 mol % charged phospholipid. In an especially preferred lipid mixture, the molar ratio of neutral phospholipid:cholesterol: charged phospholipid is 9:5:1.

The liposomes should preferably have an average particle size not greater than 200 nanometers, especially 80 to 180 nanometers.

It is generally desirable to have as high a weight ratio of α-interferon hybrid to lipid as possible consistent with liposome stability. The maximum for this weight ratio may vary depending on the nature and composition of the lipid component, but in general is likely to be about 1:200. However, it has been found that good results can be obtained with liposomes in which this ratio is from 1:400 to 1:300.

The α-interferon hybrid-containing liposomes of the invention can be prepared using known methods for the preparation of drug-containing liposomes. In accordance with the invention, a preferred method of preparing a composition of the invention as hereinbefore defined comprises removing solvent, preferably by freeze drying, from a solution of a lipid mixture as hereinbefore defined in an organic solvent to give a lipid residue, mixing the lipid residue with an aqueous medium containing the α-interferon hybrid, agitating the resulting mixture to obtain an aqueous suspension of liposomes containing entrapped α-interferon hybrid and extruding the suspension obtained, optionally after dilution with an aqueous medium, through one or more membrane filters.

Suitable organic solvents for use in the above method include alcohols, ethers, hydrocarbons, halohydrocarbons and mixtures thereof. Good results have been obtained with alcohols, especially tert-butanol.

The aqueous medium containing the α-interferon hybrid usually contains a buffer to maintain the medium at pH 7.0 to 8.0, preferably 7.5 to 7.7. Agitation of the mixture of this medium with the lipid residue may be effected in a conventional manner, for example using a Vortex mixer or by stirring at high speed. Membrane filters suitable for the extrusion of the aqueous lipsome suspension to obtain the desired particle size include conventional polycarbonate filters having a defined pore size.

The aqueous liposome suspension obtained after extrusion may be further treated to separate the liposomes from unentrapped α-interferon hybrid in a conventional manner, for example by chromatography followed by dialysis against an interferon-free aqueous phase.

For storage purposes, the liposomes may be dehydrated to powder form after their preparation. This dehydration is preferably effected in a conventional manner by lyophilisation (freeze drying) of an aqueous suspension of the liposomes. Lyophilisation is generally carried out in the presence of a cryoprotectant, preferably a sugar such as sucrose, maltose, trehalose or, especially, lactose, to give a powder comprising dehydrated liposomes in admixture with cryoprotectant. The liposomes may be reconstituted when required by treatment of the dehydrated powder in a conventional manner with water.

The pharmaceutical composition of the invention may be administered parenterally, for example intramuscularly, intradermally or, preferably, intravenously. The composition may be sterilised and may contain conventional excipients such as preservatives, stabilisers, emulsifiers, solubilisers or buffers. The daily dose may vary according to the age and weight of the patient to be treated and the state of the disease concerned.

In vivo experiments on rats with the pharmaceutical composition of the invention have shown a high degree of uptake of α-interferon hybrid by liver cells following intravenous administration, indicating the suitability of the composition for the treatment of viral liver diseases such as hepatitis B and hepatitis C.

The invention is illustrated by the following Examples.

### Example 1

A solution of a mixture of dimyristoyl phosphatidyl choline, cholesterol and dioleoyl phosphatidylserine in a molar ratio of 9:5:1 (1g) in tert-butanol (10ml) is heated to 50°C with constant stirring. Aliquots (1 ml) are transferred to 7ml glass vials, which are maintained in liquid nitrogen until their contents are frozen and then placed in a pre-cooled metal dessicator connected to a vacuum pump via a cooled solvent trap. The contents of the vials are freeze dried over 24 hours in a cool room and the resulting lipid cake is stored at -20°C.

α-Interferon hybrid rIFN-αBDBB (0.64 mg, 160 x 10⁶ IU) in phosphate-buffered (pH 7.6) aqueous 200 mM mannitol (5 ml) is added to the lipid cake at 30°C and the mixture is gently agitated on a Vortex mixer for 3 to 10 seconds. The resulting liposome suspension is diluted to 50 ml with further phosphate-buffered mannitol and extruded ten times at 30°C through polycarbonate filters having a pore size of 0.2µm under nitrogen pressure. The resulting suspension, containing liposomes with an average size of 180nm, is chromatographed on a Sepharose CL-6B column. Fractions containing the liposomes are recovered, pooled and concentrated to 50 mg/ml of lipid using ultrafiltration. The resulting suspension is dialysed overnight against an aqueous phosphate buffered 0.26 M lactose solution, then filtered, dispensed into sterile vials and freeze dried in a Lyovac GT-4 lyophiliser using a 24 hour programme.

Liposomes are reconstituted by treating the contents of the vials with sterile pyrogen-free water and chromatographed on a Sepharose CL-6B column; no leakage of the α-interferon hybrid from the liposomes is evident.

In vitro experiments using Semliki Forest Virus - infected WISH cells show that the encapsulated α-interferon hybrid has retained its antiviral activity in the reconstituted liposomes.

### Example 2

Example 1 is repeated using ¹²⁵I-labelled rIFNα-BDBB. The liposomes obtained are subjected to in vivo pharmacokinetic studies, where they are compared with the free α-interferon hybrid in a rat model. Male wistar rats are intravenously injected in a tail vein with the liposomal and free α-interferon hybrid respectively, the α-IFN hybrid dose being the same in each case. Some of the animals are sacrificed 15 minutes after injection and others are sacrificed 2 hours after injection. The radioactivity in the livers of the sacrificed animals is determined by gamma counting and the percentage of the original α-IFN hybrid dose in the liver is calculated. The results are as follows:

| | % Dose in Liver | |
|---|---|---|
| | 15 min | 2 hours |
| Liposomal α-IFN hybrid | 39.5 | 19.3 |
| Free α-IFN hybrid | 26.32 | 7.27 |

## Claims

1. A pharmaceutical composition comprising an α-interferon B/D hybrid contained in liposomes formed from a lipid mixture comprising 50 to 75 mol % neutral phospholipid, 20 to 40 mol% cholesterol and 5 to 10 mol % charged phospholipid.

2. A composition according to claim 1, in which the α-interferon hybrid is α-interferon BDBB hybrid.

3. A composition according to claim 1 or 2, in which the major component of the lipid mixture is neutral phospholipid component having a phase transition temperature of 20 to 30°C.

4. A composition according to claim 3, in which the neutral phospholipid component comprises one or more phosphatidylcholines.

5. A composition according to claim 3, in which the neutral phospholipid component is dimyristoyl phosphatidylcholine or a mixture thereof with another neutral phosphatidylcholine, said mixture having a phase transition temperature of 20 to 30°C.

6. A composition according to any of the preceding claims, in which the charged phospholipid component comprises one or more phosphatidylserines.

7. A composition according to claim 6, in which the charged phospholipid component is dioleoyl phosphatidylserine.

8. A composition according to any of the preceding claims, in which the liposomes have an average particle size up to 200 nanometers.

9. A composition according to any of the preceding claims, in which the weight ratio of the α-interferon hybrid to the lipid mixture is from 1:400 to 1:300.

10. A composition according to any of the preceding claims, in which the liposomes are in dehydrated form.

11. The use of a composition according to any of the preceding claims in the preparation of a medicament for the treatment of viral liver disease.

## Patentansprüche

1. Pharmazeutische Zusammensetzung, die ein α-Interferon B/D -Hybrid in Liposomen enthält, die aus einem Lipidgemisch gebildet wurden, das 50 bis 75 mol % neutrales Phospholipid, 20 bis 40 mol % Cholesterin und 5 bis 10 mol % geladenes Phospholipid enthält.

2. Zusammensetzung nach Anspruch 1, worin das α-Interferonhybrid das α-Interferon BDBB-Hybrid ist.

3. Zusammensetzung nach Anspruch 1 oder 2, worin die Hauptkomponente des Lipidgemisches eine neutrale Phospholipidkomponente mit einer Phasenübergangstemperatur von 20 bis 30°C ist.

4. Zusammensetzung nach Anspruch 3, worin die neutrale Phospholipidkomponente ein oder mehrere Phosphatidylcholine enthält.

5. Zusammensetzung nach Anspruch 3, worin die neutrale Phospholipidkomponente Dimyristoylphosphatidylcholin oder ein Gemisch hiervon mit einem anderen neutralen Phosphatidylcholin ist, wobei das Gemisch eine Phasenübergangstemperatur von 20 bis 30°C hat.

6. Zusammensetzung nach einem der vorangehenden Ansprüche, worin die geladene Phospholipidkomponente ein oder mehrere Phosphatidylserine enthält.

7. Zusammensetzung nach Anspruch 6, worin die geladene Phospholipidkomponente Dioleoylphosphatidylserin ist.

8. Zusammensetzung nach einem der vorangehenden Ansprüche, worin die Liposomen eine mittlere Partikelgröße von bis zu 200 Nanometern haben.

9. Zusammensetzung nach einem der vorangehenden Ansprüche, worin das Gewichtsverhältnis des α-Interferonhybrids zum Lipidgemisch 1:400 bis 1:300 beträgt.

10. Zusammensetzung nach einem der vorangehenden Ansprüche, worin die Liposomen in der dehydratisierten Form vorliegen.

11. Verwendung einer Zusammensetzung nach einem der vorangehenden Ansprüche zur Herstellung eines Arzneimittels zur Behandlung einer viralen Lebererkrankung.

## Revendications

1. Composition pharmaceutique comprenant un hybride B/D d'interféron α contenu dans des liposomes formés à partir d'un mélange lipidique comprenant de 50 à 75% en mole de phospholipide neutre, de 20 à 40% en mole de cholestérol et de 5 à 10% en mole de phospholipide chargé.

2. Composition selon la revendication 1 dans laquelle l'hybride d'interféron α est l'hybride BDBB d'interféron α.

3. Composition selon la revendication 1 ou 2, dans laquelle le composant majeur du mélange lipidique est un composant phospholipide neutre ayant une température de transition de phase comprise entre 20 et 30°C.

4. Composition selon la revendication 3 dans laquelle le composant phospholipide neutre comprend une ou plusieurs phosphatidylcholines.

5. Composition selon la revendication 3 dans laquelle le composant phospholipide neutre est la dimyristoylphosphatidylcholine ou un de ses mélanges avec une autre phosphatidylcholine neutre, ledit mélange ayant une température de transition de phase comprise entre 20 et 30°C.

6. Composition selon l'une quelconque des revendications précédentes dans laquelle le composant phospholipide chargé comprend une ou plusieurs phosphatidylsérines.

7. Composition selon la revendication 6 dans laquelle le composant phospholipide chargé est la dioléoylphosphatidylsérine.

8. Composition selon l'une quelconque des revendications précédentes dans laquelle les liposomes ont une taille particulaire moyenne allant jusqu'à 200 nanomètres.

9. Composition selon l'une quelconque des revendications précédentes dans laquelle le rapport pondéral de l'hybride d'interféron a au mélange lipidique est de 1:400 à 1:300.

10. Composition selon l'une quelconque des revendications précédentes dans laquelle les liposomes sont sous forme déshydratée.

11. Utilisation d'une composition selon l'une quelconque des revendications précédentes dans la préparation d'un médicament pour le traitement de l'hépatite virale.
